# EUROPEAN PATENT SPECIFICATION

(11) **EP 3 342 431 B1**
(45) Date of publication and mention of the grant of the patent: **27.11.2024**
(21) Application number: 18150197.4
(22) Date of filing: 03.01.2018
(51) Int. Cl.: A61L 2/28, C12Q 1/22

(54) **SELF-CONTAINED BIOLOGICAL INDICATOR**
EIGENSTÄNDIGER BIOLOGISCHER INDIKATOR
INDICATEUR BIOLOGIQUE AUTONOME

(30) Priority: 03.01.2017 US 201715397018
(43) Date of publication of application: 04.07.2018
(73) Proprietor: ASP Global Manufacturing GmbH, 8207 Schaffhausen (CH)
(72) Inventor: TRUONG, Doug Vo, Irvine, CA 92618 (US)
(74) Representative: Regimbeau

(56) References cited:
- US-A- 4 528 268
- US-A- 4 732 850
- US-A1- 2015 004 682
- US-A1- 2015 167 047
- US-B2- 8 915 413

## Description

### FIELD

The subject matter disclosed herein relates to self-contained biological sterilization indicators.

### BACKGROUND

Medical devices are typically sterilized before use in order to minimize the likelihood that a contaminated device might be used on a subject, which could cause an infection in the subject. Various sterilization techniques may be employed, such as steam, hydrogen peroxide, and vapor phase sterilization, either with or without a gas plasma and ethylene oxide (EtO). Each of these methods depends to a certain extent on the diffusion rates of the sterilization fluids, typically gases, upon the medical devices to be sterilized.

Before sterilization, medical devices are typically packaged within containers or pouches having a semi-permeable barrier that allows transmission of the sterilizing fluid-sometimes referred to as a sterilant-but prevents admission of contaminating organisms, particularly post-sterilization and until the package is opened by medical personnel. For the sterilization cycle to be efficacious, the contaminating organisms within the package must be killed because any organisms that survive the sterilization cycle could multiply and recontaminate the medical device.

Although the packaging helps prevent contamination of a sterile medical device, the packaging may increase the difficulty of achieving a successful sterilization cycle because the packaging impedes the sterilant from reaching the device or instrument contained therein. This is particularly problematic for devices and instruments that have diffusion-restricted spaces therein because these diffusion-restricted spaces reduce the likelihood that a sterilization cycle may be effective. For example, endoscopes typically have long narrow lumens into which the sterilant must diffuse in sufficient concentration for sufficient time to achieve a successful sterilization cycle.

Confirming that a sterilization cycle has been efficacious helps medical personnel avoid using a contaminated medical device on a subject. Typically, the sterilized medical device is not itself checked for contaminating organisms because such an activity would introduce other contaminating organisms to the medical device, thereby re-contaminating it. Thus, an indirect check has been developed in the form of a sterilization indicator.

A sterilization indicator is a device that may be placed alongside or in proximity to a medical device being subject to a sterilization cycle, such that the sterilization indicator is subject to the same sterilization cycle as the medical device. For instance, a biological indictor having a predetermined quantity of microorganisms possessing known resistance to the sterilant may be placed into a sterilization chamber alongside a medical device and subjected to a sterilization cycle. After the cycle is complete, the microorganisms in the biological indicator may be cultured to determine whether any of the microorganisms survived the cycle.

Certain biological indicators are referred to as being "self-contained." These biological indicators typically include a housing that contains a quantity of microorganisms and a source of growth media in a frangible container that is located near the microorganisms. Like other biological indicators, the "self-contained" biological indicator ("SCBI") may be subject to a sterilization cycle alongside medical devices. Following the cycle, the frangible container may be broken to release the growth media and culture any surviving microorganisms in situ. The SCBI may be incubated at elevated temperatures, typically around 50°C to 60°C, which encourages outgrowth of the surviving microorganisms. Incubation using commercially available products typically lasts for about twenty-four hours. During this time, while the effectiveness of the sterilization remains unconfirmed, it is desirable that medical personnel do not use the medical devices. This may cause inventory management inefficiencies for a health care provider, such as a hospital, because, for example, the medical devices should be stored while they cannot be used, perhaps requiring the health care provider to keep more medical devices in its inventory than it otherwise would to ensure a sufficient supply of medical devices. Alternatively, health care providers may use the medical devices before the incubation is completed and sterilization efficacy confirmed. However, using the medical devices before sterilization efficacy has been confirmed may expose a subject of a medical procedure to risk of infection from the medical devices.

After incubation, the SCBI is analyzed to detect the presence of microorganisms. Should any microorganisms be detected, some SCBIs are designed to incorporate a growth medium that changes color in the presence of microorganisms. If a color change is detected, the sterilization cycle may be considered to have been ineffective. Should no microorganisms be detected, the sterilization cycle may be considered to have been effective. This color change may be due to a shift in pH that occurs due to acid production by live microorganisms that metabolize a growth medium, which also contains a pH indicating dye. Other SCBIs are designed to incorporate a growth medium that includes a fluorophore whose fluorescence depends on the amount of viable microorganisms contained in the medium. For these SCBIs, a color change or change in the amount of fluorescence indicates that surviving microorganisms may have multiplied during incubation.

The frangible container of the SCBI that contains the liquid growth medium is often fabricated from glass. The glass must be sufficiently robust to avoid breakage during transportation, e.g., from the manufacturer of the SCBI to a health care provider. Such robustness, however, corresponds to a greater force required to break the ampule at the desired time by medical personnel. Accordingly, some SCBI manufacturers provide activation devices to hospital personnel to assist them in breaking the ampule.
US2015167047A1 relates to a biological sterilization indicator, BI, and a method of using same for assaying the lethality of a sterilization process. The BI can include a housing, which can include a first portion, and a second portion, which can be movable with respect to the first portion between a first and second position. The BI can further include a frangible container comprising a liquid. The BI can further include a spore reservoir and a projection positioned in the housing. The projection can be configured to fracture the container when the second portion of the housing is moved from the first position to the second position. The method can include maintaining a minimal cross-sectional area of space around the container when the second portion of the housing is in the first position, and fracturing the container in response to moving the second portion between the first and second positions.
US2015004682A1 relate a biological sterilization indicator, BI, and method of using same. The BI can include a housing, and a container positioned in the housing. The container can contain a liquid and at least a portion of the container can be frangible. The BI can further include a first chamber and a second chamber. The second chamber can include at least one source of biological activity. The BI can further include a first fluid path positioned to fluidly couple the first chamber and the second chamber, and a second fluid path positioned to allow displaced gas to move out of the second chamber. The method can include moving displaced gas out of the second chamber via the second fluid path as a sterilant is moved into the second chamber via the first fluid path and/or as the liquid is moved into the second chamber via the first fluid path.
US2013032623A1 relates to a device for opening an ampoule, said device comprising a substantially hollow cylindrical housing having an interior for accommodating an ampoule. The device also comprises a rotary element having a proximal section, to which a break-off section extending in the distal direction is connected. The break-off section is flexible in the radial direction and/or is pivotably connected to the proximal section in the radial direction. The rotary element can be rotated relative to the housing about the longitudinal axis in an actuating direction from an initial position to an intermediate position.; In the area of the side wall, the housing has a first guiding structure, which is designed in such a way that the first guiding structure presses the break-off section inward during a rotation from the initial position to the intermediate position so that the break-off section exerts a radial shear force on the ampoule head of an ampoule accommodated in the interior in order to break the ampoule head off of the ampoule body. US4732850A relates to a biological indicator is made of a vial which contains a spore dot, a nutrient solution in a frangible ampoule, and means for positioning the spore dot and for breaking the ampoule to release the nutrient solution when the cap of the vial is fully pressed on. The nutrient solution may contain a pH indicator to determine whether all active spores on the strip were killed by a sterilization process which takes place prior to breaking the ampoule.

### SUMMARY

In a first aspect of the invention there is provided a self-contained biological sterilization indicator that includes a housing and an ampule disposed within the housing, the ampule having a first dome, a second dome and a sidewall. A cap is coupled to the housing. The cap includes a projection that is coupled to the ampule by a friction fit such that a central longitudinal axis of the ampule and a central longitudinal axis of the housing are aligned. An insert is disposed within the housing. The insert includes a well within which at least a portion of the bottom dome of the ampule is disposed. The insert also includes a ramp that is angled at least 5 degrees relative to the central longitudinal axis of the ampule and in contact with the second dome of the ampule, the ramp being configured to apply a first reaction force at the point of contact with the second dome of the ampule when a compressive force is applied between the cap and the housing along the direction of the central longitudinal axis of the housing. The insert also includes a stress concentrator disposed adjacent to the sidewall of the ampule, the stress concentrator being configured to apply a second reaction force against the sidewall of the ampule when the first reaction force is applied, thereby facilitating breakage of the ampule.

In some embodiments, the ramp and the stress concentrator are configured to restrict movement of the ampule away from the cap along the central longitudinal axis of the ampule. In some embodiments the stress concentrator has a pointed tip. In some embodiments the stress concentrator has a rounded tip. In some embodiments the stress concentrator may be disposed at least approximately 5mm above the second dome. In some embodiments the stress concentrator may be disposed at least approximately 10 mm above the second dome. In some embodiments the stress concentrator spans an arc of at least approximately 5 degrees. In some embodiments the stress concentrator spans an arc of at least 15 degrees. In some embodiments the ramp spans an arc of at least approximately 30 degrees. In some embodiments the ramp spans an arc of at least approximately 50 degrees. In some embodiments the insert has a circular shape such that a midpoint of an arc spanned by the ramp and a midpoint of an arc spanned by the stress concentrator are collinear with a diameter of the insert. In some embodiments the ampule does not contact the housing. In some embodiments the stress concentrator contacts the sidewall of the ampule.

Also disclosed herein are steps that may be performed to activate the self-contained biological sterilization indicator. These steps include: a) generating a first reaction force between the second dome of the ampule and the ramp wherein a component of the first reaction force is directed transverse to a central longitudinal axis of the ampule; b) generating a second reaction force between the sidewall of the ampule and a tip of the stress concentrator in response to the component of the first reaction force; and c) breaking the ampule. The step of breaking the ampule may include initiating a crack at a point of contact between the stress concentrator and the sidewall of the ampule. Further, the first reaction force may be generated by applying a compressive force between the cap and the housing of the self-contained biological sterilization indicator.

The insert of the self-contained biological sterilization indicator may include no more than one stress concentrator. The angled surface of the self-contained biological sterilization indicator may be a feature of the insert. The insert may be disposed within the housing of the self-contained biological sterilization indicator. The ampule may rest upon the angled surface before the reaction force is generated. The cap may be coupled to the housing and it may also be coupled to the ampule by a friction fit. The ampule cannot contact the housing.

As used herein, the term "surface" should be understood as a feature of an object that forms a boundary of the object.

As used herein, the term "wall" should be understood as a feature of an object that forms at least a portion of a side, top, or bottom, of that object. A wall is an example of a surface.

As used herein, the term "insert" should be understood as an object that is disposed within a space or cavity defined by another object.

As used herein, the terms "arm," "finger," "leg," and "projection" should each be understood as an elongate member of an object that originates at and extends away from another feature of that object.

As used herein, the term "carrier" should be understood as an object upon which microorganisms and/or enzymes have been disposed.

As used herein, the term "reaction force" should be understood as a force generated by an object in response to another force on the object, where at least a component of the reaction force points in a direction opposite to the direction of the another force.

As used herein, the term "stress concentrator" should be understood as a feature that includes a surface area configured to exert a reaction force against an object, where the surface area configured to exert the reaction force is less than a surface area of the object upon which another force is exerted.

As used herein, the term "friction fit" should be understood as a coupled relationship between two or more surfaces that is achieved by friction.

### BRIEF DESCRIPTION OF THE DRAWINGS

While the specification concludes with claims which particularly point out and distinctly claim the subject matter described herein, it is believed the subject matter will be better understood from the following description of certain examples taken in conjunction with the accompanying drawings, in which like reference numerals identify the same elements and in which:
Fig. 1 depicts a side section view of an SCBI;
Fig. 2 depicts an isometric exploded view of the SCBI;
Fig. 3 depicts a top view of an insert of the SCBI; and
Fig. 4 depicts a detail view for a portion of the section view of Fig. 1.

### DETAILED DESCRIPTION

The following description sets forth certain illustrative examples of the claimed subject matter. Other examples, features, aspects, embodiments, and advantages of the technology should become apparent to those skilled in the art from the following description. Accordingly, the drawings and descriptions should be regarded as illustrative in nature.

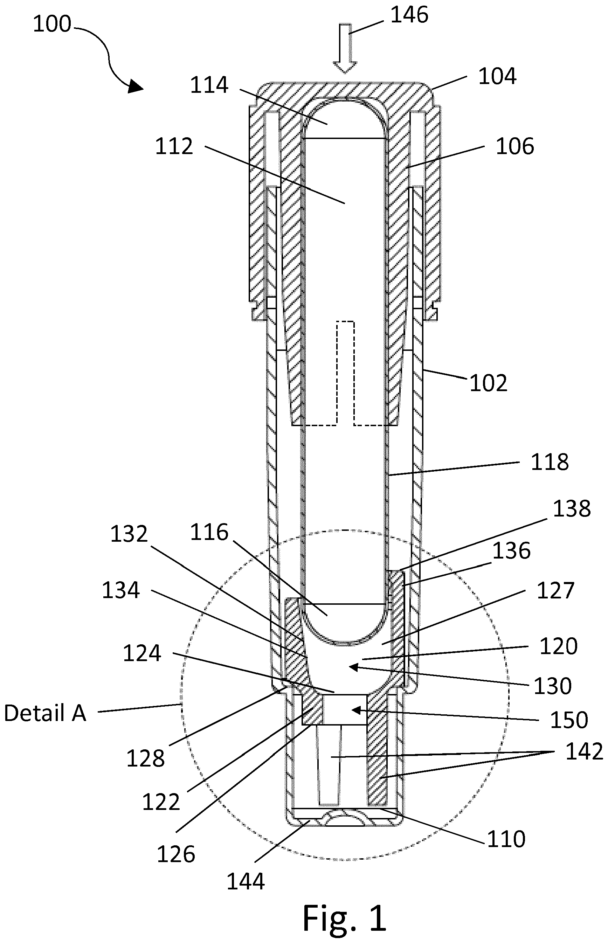

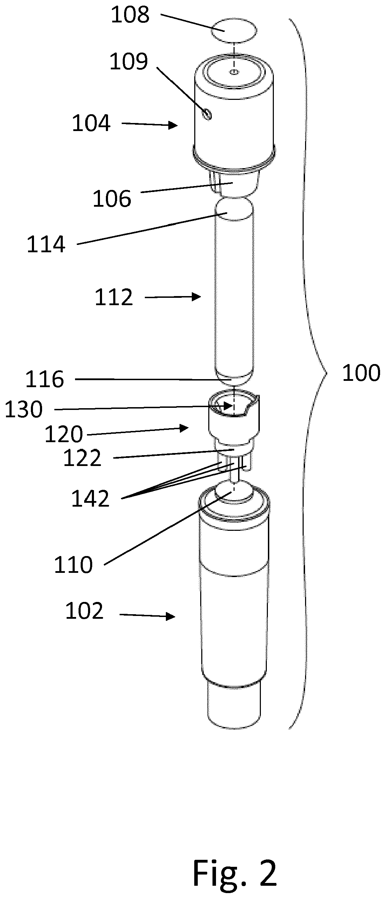

Referring to Figs. 1 and 2, a self-contained biological indicator ("SCBI") 100 is shown. SCBI 100 includes a housing 102 and a cap 104 coupled thereto. Cap 104 includes a projection 106 that has a planar, angled, arcuate, annular, or conical shape, or some combination thereof. Cap 104 may further include a chemical indicator 108 that changes color when exposed to, e.g., a chemical sterilant such as hydrogen peroxide. Cap 104 may also include one or more through-holes 109, to assist in the passage of gasses (e.g., air or sterilant) into or out from the SCBI. Cap 104 is coupled relative to housing 102 in a first position and is movable from the first position to a second position. In the first position (shown in Figs. 1 and 2), Cap 104 is coupled to housing 102 in a manner in which gases (e.g., air or sterilant) may move from the surrounding environment and into the SCBI, or vice versa. In this position, any through-holes in cap 104 are disposed above housing 102 such that the inside of housing 102 is in fluid communication with the surrounding environment, which permits introduction and withdrawal of sterilant into and from SCBI 100. Cap 104 may be depressed to move it into the second position relative to housing 102. In this second position, through-holes 109 are disposed below a top end of housing 102, which causes a tight fitting relationship between housing 102 and cap 104, and blocks the through holes, effectively sealing off the inside of the SCBI 100 from the surrounding environment.

SCBI 100 also includes a source of microorganisms or active enzymes, such as carrier 110, which is impregnated with bacterial spores, other forms of bacteria (e.g., vegetative), and/or active enzymes. Spores from Bacillus, Geobacillus, and Clostridia species are often used to monitor sterilization processes utilizing saturated steam, hydrogen peroxide, dry heat, gamma irradiation and ethylene oxide. Accordingly, carrier 110 may be impregnated with spores from Bacillus, Geobacillus, and/or Clostridia species. Carrier 110 may be water-absorbent and may be formed of filter paper. Sheet-like materials such as cloth, nonwoven polypropylene, rayon or nylon, and microporous polymeric materials may also be used. Non-water absorbent materials are also appropriate for use, such as metals (e.g., aluminum or stainless steel), glass (e.g., glass beads or glass fibers), porcelain, or plastic. Additionally, carrier 110 can be constructed of a combination of the aforementioned materials. In some embodiments, carrier 110 may have a thickness of approximately 0.1 to 0.5 millimeters.

SCBI 100 also includes an ampule 112, having a first end 114, a second end 116, and a sidewall 118. Sidewall 118 is substantially cylindrical and may have an elliptical or circular cross section. First end 114 and second end 116 dome are disposed at opposite ends of sidewall 118, and may have the form of a hemiellipsoid or hemisphere. Accordingly, first end 114 may be referred to as first dome 114 and second end 116 may be referred to as second dome 116. Ampule 112 contains a liquid growth medium. The growth medium should be capable of promoting growth of any viable microorganisms disposed on carrier 110. Growth may be accelerated by incubation at elevated temperatures, e.g., between approximately 50°C and 60 °C. Ampule 112 may be fabricated from a frangible or brittle material such as glass or plastic.

SCBI 100 may also include an insert 120, which is shown in FIGs. 1-4. Insert 120 may include a platform 122 having a top surface 124 and a bottom surface 126. Insert 120 also includes a sidewall 127. Sidewall 127 of platform 122 may rest upon a support surface 128, which may be integrally formed as part of housing 102. Sidewall 127 and top surface 124 of platform 122 together define a well 130, which is configured to receive second end 116 of ampule 112. Platform 122 defines a bore 150 therethrough, through which the liquid growth medium may pass upon breakage of the ampule.

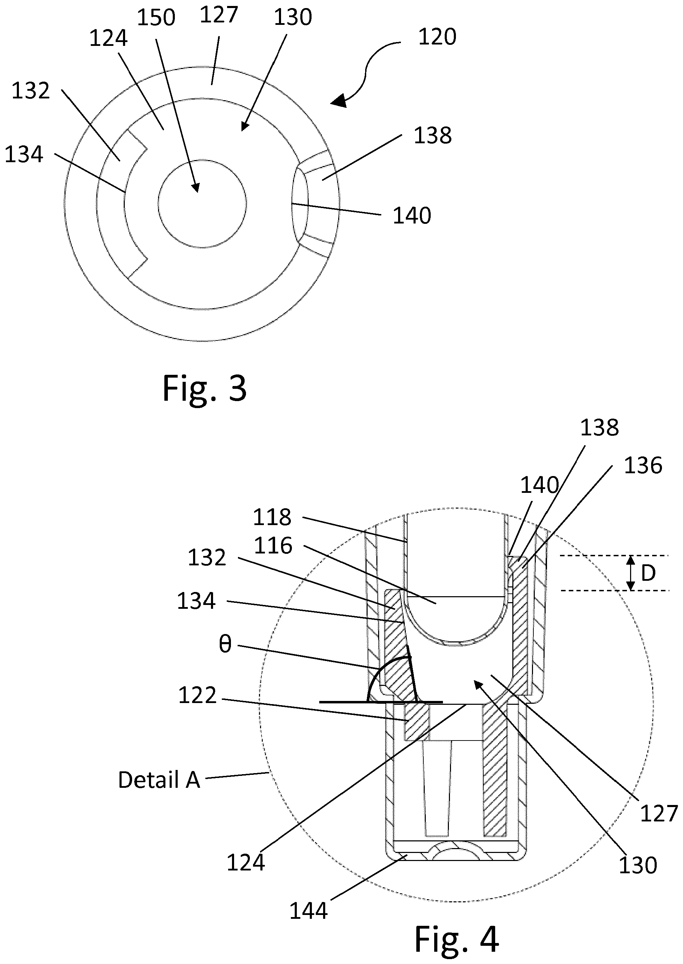

Insert 120 further includes a ramp 132. Ramp 132 is disposed upon or formed as an integral feature of sidewall 127. Ramp 132 includes an angled surface or ramp surface 134. Ramp surface 134 and top surface 124 of platform 122 are disposed in an angular relationship. As shown in Fig. 4, ramp surface 134 is disposed at θ degrees with respect to platform top surface 124. θ may be equal to approximately 85 degrees, 80 degrees, 75 degrees, 70 degrees, or less.

Insert 120 also includes a finger (arm) 136 that extends above well 130 of insert 120. Finger 136 is disposed opposite ramp 132. Disposed upon finger 136, facing ramp 132, is a stress concentrator 138. Stress concentrator 138 may include a tip 140. Tip 140 may be pointed. It may have a triangular configuration. Tip 140 may alternatively have a rounded configuration. Tip 140 is spaced a distance D above sidewall 127. D may be equal to approximately between 1 mm and 25 mm, between approximately 1 mm and 15 mm, between approximately 1 mm and 5 mm, or it may be between approximately 1-3 mm.

Ramp 132 is disposed along sidewall 127 within well 130 while finger 136 extends upward from sidewall 127. Accordingly, both ramp 132 and finger 136 may have arcuate configurations having curvatures similar to the curvature of sidewall 127. In some embodiments, a midpoint of the arc of ramp 132 and a midpoint of the arc of finger 136 are collinear with a diameter of insert 120. In some embodiments, the arc of ramp 132 spans at least approximately 30 degrees. In some embodiments, the arc of ramp 132 spans at least approximately 50 degrees. In some embodiments, the arc of finger 136 spans at least approximately 15 degrees. In some embodiments, the arc of finger 136 spans at least approximately 30 degrees.

Insert 120 may also include a leg (or legs) 142 that originates from bottom surface 126 of platform 122 and extends away therefrom. Leg 142 has a maximum length equal to the distance between support surface 128 and a bottom wall 144 of housing 102. As shown in Fig. 4, leg 142 has a length that is somewhat less than the distance between support surface128 and bottom wall 144. Leg 142 may have a length that is approximately 0.1 to 1 millimeter less than the distance between support surface 128 and bottom wall 144. Insert 120 may include multiple instances of leg 142. For example, insert 120 may include three instances of leg 142. Leg or legs 142 function to maintain carrier 110 against bottom wall 144 to help prevent carrier 110 from becoming damaged or dislodged during transportation.

SCBI 100 may be assembled according to the following steps. First, housing 102 is provided. Second, carrier 110 is placed into housing 102 such that it rests upon bottom wall 144 of housing 102. Third, insert 120 is placed into housing 102 such that sidewall 127 of platform 122 rests upon support surface 128. Alternatively, not shown, in some configurations lacking a support surface 128, insert 120 may rest directly upon bottom wall 142 and may be in at least partial contact with carrier 110. Fourth, ampule 112 is inserted into housing 102 such that second end 116 contacts ramp 132. Finally, cap 104 is coupled to housing 102 and ampule 112. Projection 106 has approximately the same diameter as ampule 112 such that a friction fit is formed between ampule 112 and projection 106. So assembled, central longitudinal axes of ampule 112, housing 102, cap 104, and insert 120 are coaxial or substantially coaxial. Other assembly procedures may be performed to achieve the same configuration of SCBI 100.

The configuration of SCBI 100, and in particular, the relationships between its various components, maintain ampule 112 in a stationary state, which helps prevent premature breakage of ampule 112, e.g., during transportation from a manufacturing facility to a healthcare facility. For example, cap 104, ramp 132 and stress concentrator 138 prevent ampule 112 from moving away from cap 104 or moving in a lateral direction within housing 102. In some configurations, ampule 112 may contact tip 140. In other configurations, there may be spacing between ampule 112 and tip 140.

Because the central longitudinal axes of ampule 112 and insert 120 are coaxial, ramp 132 is disposed at an angle ϕ to ampule 112 where ϕ equals 90 degrees minus θ. Accordingly, ϕ may be equal to approximately 5 degrees, 10 degrees, 15 degrees, 20 degrees, or more.

Following a sterilization procedure, an SCBI may be activated and monitored to determine whether a sterilization cycle was effective. To activate SCBI 100, a compressive force 146 is applied between housing 102 and cap 104. This compressive force is resisted by ampule 112 because ampule 112 is in contact with ramp 132 of insert 120 and insert 120 is in contact with, e.g., support 128 of housing 102. When the compressive force applied to cap 104 is greater than a breakage force ampule 112 can withstand, ampule 112 will break. Once ampule 112 is broken, cap 104 moves to its second position and growth medium is released to immerse carrier 110.

The specific breakage mechanism of ampule 112 provided by SCBI 100 lowers the magnitude of the compressive force that must be applied to cap 104 to break ampule 112.. Specifically, the compressive downward force applied to cap 104 causes a first reaction force at the point of contact between second end 116 of ampule 112 and ramp 132 of insert 120. This reaction force is perpendicular to ramp surface 134. Accordingly, this reaction force includes a component that is substantially parallel to the central longitudinal axis of ampule 112 and a component that is substantially perpendicular or transverse to the central longitudinal axis of ampule 112. The transverse component causes sidewall 118 of ampule 112 to press against stress concentrator 138, or more specifically tip 140 of stress concentrator 138, which causes a second reaction force against sidewall 118 that is transverse to the central longitudinal axis of ampule 112. The magnitude of this reaction force may be approximated as the magnitude of the force applied to the cap multiplied by tan(cp). As the magnitude of the compressive force applied to cap 104 increases, so too does the magnitude of the first reaction force and the second reaction force until the second reaction force becomes greater than the ampule can withstand, which causes the ampule to break. Because the second reaction force is applied laterally to sidewall 118, the breakage is initiated at the point of contact between stress concentrator 138 and sidewall 118. Initial cracks may be formed at or near this point of contact before the ampule breaks. When ampule 112 is fabricated from glass, formation of the initial crack or cracks is promptly followed by the glass shattering into many shards. These shards collect upon insert 120, allowing the growth medium to flow through and alongside insert 120 to immerse carrier 110.

Before activation of SCBI 100, first end 114 of ampule 112 is maintained within projection 106 of cap 104 and second end 116 of ampule 112 is maintained within well 130, between ramp 132 and stress concentrator 138. This configuration has two beneficial purposes. First, this configuration minimizes the magnitude of the compressive force that must be applied to the cap to break the ampule because the compressive force presses ampule 112 against ramp 132, which removes the collinear relationship between the longitudinal axis of ampule 112 and the longitudinal axis of insert 120. Accordingly, stress concentrator 138 presses against sidewall 118 in an asymmetric manner, which increases the stresses in ampule 112, in part by avoiding generation of internal stresses that cancel each other, thereby facilitating breakage of ampule 112. Second, this configuration restricts movement of the ampule within the SCBI, which minimizes rattling and jostling of ampule 112 within SCBI 100 and helps avoid premature breakage of the ampule, e.g., during transportation.

It should be understood that any of the examples and/or embodiments described herein may include various other features in addition to or in lieu of those described above. The teachings, expressions, embodiments, examples, etc. described herein should not be viewed in isolation relative to each other. Various suitable ways in which the teachings herein may be combined should be readily apparent to those of ordinary skill in the art in view of the teachings herein.

Having shown and described exemplary embodiments of the subject matter contained herein, further adaptations of the methods and systems described herein may be accomplished by appropriate modifications without departing from the scope of the claims. Some such modifications should be apparent to those skilled in the art. For instance, the examples, embodiments, geometries, materials, dimensions, ratios, steps, and the like discussed above are illustrative. Accordingly, the claims should not be limited to the specific details of structure and operation set forth in the written description and drawings.

## Claims

1. A self-contained biological sterilization indicator (100) comprising:
(a) a housing (102);
(b) an ampule (112) disposed within the housing, the ampule having a first dome (114), a second dome (116), and a sidewall (118);
(c) a cap (104) coupled to the housing, the cap having a projection (106) coupled to the ampule by a friction fit such that a central longitudinal axis of the ampule and a central longitudinal axis of the housing are aligned; and
(d) an insert (120) disposed within the housing, the insert having
(i) a well (130) within which at least a portion of the second dome is disposed;
(ii) a ramp (132) angled at least 5 degrees relative to the central longitudinal axis of the ampule and in contact with the second dome of the ampule, the ramp being configured to apply a first reaction force at the point of contact with the second dome of the ampule when a compressive force is applied between the cap and the housing along the direction of the central longitudinal axis of the housing; and
(iii) a stress concentrator (138) disposed adjacent to the sidewall of the ampule, the stress concentrator being configured to apply a second reaction force against the sidewall (118) of the ampule when the first reaction force is applied, thereby facilitating breakage of the ampule (112).

2. The self-contained biological sterilization indicator of claim 1 wherein the insert further comprises an arm (136) extending above the well, the stress concentrator being disposed on the arm.

3. The self-contained biological sterilization indicator of claim 1 wherein the ramp and the stress concentrator are configured to restrict movement of the ampule away from the cap along the central longitudinal axis of the ampule.

4. The self-contained biological sterilization indicator of claim 1 wherein the stress concentrator has a pointed tip.

5. The self-contained biological sterilization indicator of claim 1 wherein the stress concentrator has a rounded tip.

6. The self-contained biological sterilization indicator of claim 1 wherein the stress concentrator is disposed at least 5mm above the second dome.

7. The self-contained biological sterilization indicator of claim 6 wherein the stress concentrator is disposed at least 10 mm above the second dome.

8. The self-contained biological sterilization indicator of claim 1 wherein the stress concentrator spans an arc of at least 5 degrees.

9. The self-contained biological sterilization indicator of claim 1 wherein the stress concentrator spans an arc of at least 15 degrees.

10. The self-contained biological sterilization indicator of claim 9 wherein the stress concentrator spans an arc of at least 30 degrees.

11. The self-contained biological sterilization indicator of claim 1 wherein the ramp spans an arc of at least 30 degrees.

12. The self-contained biological sterilization indicator of claim 11 wherein the ramp spans an arc of at least 50 degrees.

13. The self-contained biological sterilization indicator of claim 1 wherein the insert has circular shape and wherein a midpoint of an arc spanned by the ramp and a midpoint of an arc spanned by the stress concentrator are collinear with a diameter of the insert.

14. The self-contained biological sterilization indicator of claim 1 wherein the ampule does not contact the housing.

15. The self-contained biological sterilization indicator of claim 1 wherein the stress concentrator contacts the sidewall of the ampule.

16. A method of activating the self-contained biological sterilization indicator of claim 1, comprising:
(a) generating a first reaction force between the second dome of the ampule and the ramp wherein a component of the first reaction force is directed transverse to a central longitudinal axis of the ampule; and
(b) generating a second reaction force between the sidewall of the ampule and a tip of the stress concentrator in response to the component of the first reaction force; and
(c) breaking the ampule.

17. The method of claim 16 wherein the step of breaking the ampule includes initiating a crack at a point of contact between the stress concentrator and the sidewall of the ampule.

18. The method of claim 17 wherein the stress concentrator is configured to contact the sidewall of the ampule and the insert includes no more than one stress concentrator.

19. The method of claim 18 wherein the ampule cannot contact the housing of the self-contained biological sterilization indicator.

20. The method of claim 19 further comprising applying a compressive force between the cap and the housing to generate the first reaction force.

## Patentansprüche

1. Eigenständiger biologischer Sterilisationsindikator (100), umfassend:
(a) ein Gehäuse (102);
(b) eine Ampulle (112), die innerhalb des Gehäuses angeordnet ist, wobei die Ampulle eine erste Kuppel (114), eine zweite Kuppel (116) und eine Seitenwand (118) aufweist;
(c) eine Kappe (104), die an das Gehäuse gekoppelt ist, wobei die Kappe einen Vorsprung (106) aufweist, der derart durch Reibschluss an die Ampulle gekoppelt ist, dass eine mittlere Längsachse der Ampulle und eine mittlere Längsachse des Gehäuses fluchten; und
(d) einen Einsatz (120), der innerhalb des Gehäuses angeordnet ist, wobei der Einsatz aufweist:
(i) eine Mulde (130), in der mindestens ein Abschnitt der zweiten Kuppel angeordnet ist;
(ii) eine Rampe (132), die um mindestens 5 Grad in Bezug auf die mittlere Längsachse der Ampulle gewinkelt ist und mit der zweiten Kuppel der Ampulle in Berührung steht, wobei die Rampe dazu ausgestaltet ist, eine erste Reaktionskraft am Berührungspunkt mit der zweiten Kuppel der Ampulle anzuwenden, wenn eine Druckkraft zwischen der Kappe und dem Gehäuse entlang der Richtung der mittleren Längsachse des Gehäuses angewandt wird; und
(iii) einen Spannungskonzentrator (138), der benachbart zu der Seitenwand der Ampulle angeordnet ist, wobei der Spannungskonzentrator dazu ausgestaltet ist, eine zweite Reaktionskraft gegen die Seitenwand (118) der Ampulle anzuwenden, wenn die erste Reaktionskraft angewandt wird, wodurch der Bruch der Ampulle (112) erleichtert wird.

2. Eigenständiger biologischer Sterilisationsindikator nach Anspruch 1, wobei der Einsatz ferner einen Arm (136) umfasst, der sich über der Mulde erstreckt, wobei der Spannungskonzentrator auf dem Arm angeordnet ist.

3. Eigenständiger biologischer Sterilisationsindikator nach Anspruch 1, wobei die Rampe und der Spannungskonzentrator dazu ausgestaltet sind, Bewegung der Ampulle weg von der Kappe entlang der mittleren Längsachse der Ampulle einzuschränken.

4. Eigenständiger biologischer Sterilisationsindikator nach Anspruch 1, wobei der Spannungskonzentrator eine spitze Spitze aufweist.

5. Eigenständiger biologischer Sterilisationsindikator nach Anspruch 1, wobei der Spannungskonzentrator eine abgerundete Spitze aufweist.

6. Eigenständiger biologischer Sterilisationsindikator nach Anspruch 1, wobei der Spannungskonzentrator mindestens 5 mm über der zweiten Kuppel angeordnet ist.

7. Eigenständiger biologischer Sterilisationsindikator nach Anspruch 6, wobei der Spannungskonzentrator mindestens 10 mm über der zweiten Kuppel angeordnet ist.

8. Eigenständiger biologischer Sterilisationsindikator nach Anspruch 1, wobei der Spannungskonzentrator einen Bogen von mindestens 5 Grad überspannt.

9. Eigenständiger biologischer Sterilisationsindikator nach Anspruch 1, wobei der Spannungskonzentrator einen Bogen von mindestens 15 Grad überspannt.

10. Eigenständiger biologischer Sterilisationsindikator nach Anspruch 9, wobei der Spannungskonzentrator einen Bogen von mindestens 30 Grad überspannt.

11. Eigenständiger biologischer Sterilisationsindikator nach Anspruch 1, wobei die Rampe einen Bogen von mindestens 30 Grad überspannt.

12. Eigenständiger biologischer Sterilisationsindikator nach Anspruch 11, wobei die Rampe einen Bogen von mindestens 50 Grad überspannt.

13. Eigenständiger biologischer Sterilisationsindikator nach Anspruch 1, wobei der Einsatz eine Kreisform aufweist und wobei ein Mittelpunkt eines Bogens, der von der Rampe überspannt wird, und ein Mittelpunkt eines Bogens, der von dem Spanungskonzentrator überspannt wird, kollinear mit einem Durchmesser des Einsatzes sind.

14. Eigenständiger biologischer Sterilisationsindikator nach Anspruch 1, wobei die Ampulle das Gehäuse nicht berührt.

15. Eigenständiger biologischer Sterilisationsindikator nach Anspruch 1, wobei der Spannungskonzentrator die Seitenwand der Ampulle berührt.

16. Verfahren zum Aktivieren des eigenständigen biologischen Sterilisationsindikators nach Anspruch 1, umfassend:
(a) Erzeugen einer ersten Reaktionskraft zwischen der zweiten Kuppel der Ampulle und der Rampe, wobei eine Komponente der ersten Reaktionskraft quer zu einer mittleren Längsachse der Ampulle gerichtet ist; und
(b) Erzeugen einer zweiten Reaktionskraft zwischen der Seitenwand der Ampulle und einer Spitze des Spannungskonzentrators als Reaktion auf die Komponente der ersten Reaktionskraft; und
(c) Brechen der Ampulle.

17. Verfahren nach Anspruch 16, wobei der Schritt des Brechens der Ampulle das Auslösen eines Risses an einem Berührungspunkt zwischen dem Spannungskonzentrator und der Seitenwand der Ampulle umfasst.

18. Verfahren nach Anspruch 17, wobei der Spannungskonzentrator dazu ausgestaltet ist, die Seitenwand der Ampulle zu berühren, und der Einsatz nicht mehr als einen Spannungskonzentrator umfasst.

19. Verfahren nach Anspruch 18, wobei die Ampulle das Gehäuse des eigenständigen biologischen Sterilisationsindikators nicht berühren kann.

20. Verfahren nach Anspruch 19, das ferner das Anwenden einer Druckkraft zwischen der Kappe und dem Gehäuse umfasst, um die erste Reaktionskraft zu erzeugen.

## Revendications

1. Un indicateur de stérilisation biologique autonome (100) comprenant :
(a) un boîtier (102) ;
(b) une ampoule (112) disposée à l'intérieur du boîtier, l'ampoule ayant un premier dôme (114), un second dôme (116) et une paroi latérale (118) ;
(c) un capuchon (104) couplé au boîtier, le capuchon ayant une projection (106) couplée à l'ampoule par un ajustement par friction de sorte qu'un axe longitudinal central de l'ampoule et un axe longitudinal central du boîtier soient alignés ; et
(d) un insert (120) disposé à l'intérieur du boîtier, l'insert ayant
(i) un puit (130) dans lequel est disposée au moins une partie du second dôme ;
(ii) une rampe (132) inclinée d'au moins 5 degrés par rapport à l'axe longitudinal central de l'ampoule et en contact avec le second dôme de l'ampoule, la rampe étant configurée pour appliquer une première force de réaction au point de contact avec le second dôme de l'ampoule lorsqu'une force de compression est appliquée entre le capuchon et le boîtier le long de la direction de l'axe longitudinal central du boîtier ; et
(iii) un concentrateur de contrainte (138) disposé à côté de la paroi latérale de l'ampoule, le concentrateur de contrainte étant configuré pour appliquer une seconde force de réaction contre la paroi latérale (118) de l'ampoule lorsque la première force de réaction est appliquée, facilitant ainsi la rupture de l'ampoule (112).

2. L'indicateur de stérilisation biologique autonome selon la revendication 1, dans lequel l'insert comprend en outre un bras (136) s'étendant au-dessus du puits, le concentrateur de contrainte étant disposé sur le bras.

3. L'indicateur de stérilisation biologique autonome selon la revendication 1, dans lequel la rampe et le concentrateur de contrainte sont configurés pour limiter le mouvement de l'ampoule par rapport au bouchon le long de l'axe longitudinal central de l'ampoule.

4. L'indicateur de stérilisation biologique autonome selon la revendication 1, dans lequel le concentrateur de contrainte a un bout pointu.

5. L'indicateur de stérilisation biologique autonome selon la revendication 1, dans lequel le concentrateur de contrainte a un bout arrondi.

6. L'indicateur de stérilisation biologique autonome selon la revendication 1, dans lequel le concentrateur de stress est disposé à au moins 5 mm au-dessus du second dôme.

7. L'indicateur de stérilisation biologique autonome selon la revendication 6, dans lequel le concentrateur de stress est disposé à au moins 10 mm au-dessus du second dôme.

8. L'indicateur de stérilisation biologique autonome selon la revendication 1, dans lequel le concentrateur de contrainte s'étend sur un arc d'au moins 5 degrés.

9. L'indicateur de stérilisation biologique autonome selon la revendication 1, dans lequel le concentrateur de contrainte s'étend sur un arc d'au moins 15 degrés.

10. L'indicateur de stérilisation biologique autonome selon la revendication 9, dans lequel le concentrateur de contrainte s'étend sur un arc d'au moins 30 degrés.

11. L'indicateur de stérilisation biologique autonome selon la revendication 1, dans lequel la rampe s'étend sur un arc d'au moins 30 degrés.

12. L'indicateur de stérilisation biologique autonome selon la revendication 11, dans lequel la rampe s'étend sur un arc d'au moins 50 degrés.

13. L'indicateur de stérilisation biologique autonome selon la revendication 1, dans lequel l'insert a une forme circulaire et dans lequel le point médian d'un arc couvert par la rampe et le point médian d'un arc couvert par le concentrateur de contrainte sont colinéaires avec le diamètre de l'insert.

14. L'indicateur de stérilisation biologique autonome selon la revendication 1, dans lequel l'ampoule n'entre pas en contact avec le boîtier.

15. L'indicateur de stérilisation biologique autonome selon la revendication 1, dans lequel le concentrateur de contrainte est en contact avec la paroi latérale de l'ampoule.

16. méthode d'activation de l'indicateur de stérilisation biologique autonome selon la revendication 1, comprenant
(a) générer une première force de réaction entre le second dôme de l'ampoule et la rampe, une composante de la première force de réaction étant dirigée transversalement à l'axe longitudinal central de l'ampoule ; et
(b) générer une seconde force de réaction entre la paroi de l'ampoule et une pointe du concentrateur de contrainte en réponse à la composante de la première force de réaction ; et
(c) briser l'ampoule.

17. La méthode décrite selon la revendication 16, dans laquelle l'étape consistant à briser l'ampoule consiste à faire apparaitre une fissure à un point de contact entre le concentrateur de contrainte et la paroi latérale de l'ampoule.

18. La méthode selon la revendication 17, dans laquelle le concentrateur de contrainte est configuré pour toucher la paroi latérale de l'ampoule et que l'insert ne comporte pas plus d'un concentrateur de contrainte.

19. La méthode selon la revendication 17, dans laquelle l'ampoule ne peut pas entrer en contact avec le boîtier de l'indicateur de stérilisation biologique autonome.

20. La méthode selon la revendication 19 comprenant en outre l'application d'une force de compression entre le capuchon et le boîtier pour générer la première force de réaction.
